# EUROPEAN PATENT SPECIFICATION

(11) **EP 1 181 042 B1**
(45) Date of publication and mention of the grant of the patent: **14.11.2007**
(21) Application number: 00929587.4
(22) Date of filing: 26.05.2000
(51) Int. Cl.: A61K 38/18

(54) **NEUROTROPHIC FACTORS IN THE TREATMENT OF PERIPHERAL NERVE DYSFUNCTION OF PELVIC AREA**
NEUROTROPHE FAKTOREN ZUR BEHANDLUNG EINER DYSFUNKTION PERIPHERER NERVEN IM BECKENBEREICH
FACTEURS NEUTROPHIQUES UTILES DANS LE TRAITEMENT DU DYSFONCTIONNEMENT DU SYSTEME NERVEUX PERIPHERIQUE DE LA REGION PELVIENNE

(30) Priority: 27.05.1999 FI 991197
(43) Date of publication of application: 27.02.2002
(73) Proprietor: Licentia Ltd., 00130 Helsinki (FI)
(72) Inventor: SAARMA, Mart, FIN-00570 Helsinki (FI); LAURIKAINEN, Antti, Mikael, FIN-00760 Helsinki (FI); HILTUNEN, Jukka, Olavi, FIN-00350 Helsinki (FI); AIRAKSINEN, Matti, Sakari, FIN-00710 Helsinki (FI); KLINGE, Erik, Martin, FIN-00100 Helsinki (FI)
(74) Representative: Lönnqvist, Gunnel Solveig Kristina
(86) International application number: PCT/FI2000/000476
(87) International publication number: WO 2000/072871

(56) References cited:
- WO-A1-92/20365
- WO-A1-97/33911
- WO-A1-98/02178
- WO-A1-98/33529
- WO-A1-98/36072
- DATABASE MEDLINE [Online] DIALOG INFORMATION SERVICES, FILE 155 LAURIKAINEN A. ET AL.: 'Neurturin is a neurotrophic factor for penile parasympathetic neurons in adult rat', XP002908750 Retrieved from Dialog, accession no. 10634485 Database accession no. 20233899 & JOURNAL OF NEUROBIOLOGY vol. 43, no. 2, May 2000, (USA), pages 198 - 205
- DATABASE MEDLINE [Online] DIALOG INFORMATION SERVICES, FILE 155 BURGERS J.K. ET AL.: 'Nerve growth factor, nerve grafts and amniotic membrane grafts restore erectile function in rats', XP002905393 Retrieved from Dialog, accession no. 07412543 Database accession no. 91311798 & JOURNAL OF UROLOGY vol. 146, no. 2, August 1991, (USA), pages 463 - 468
- DATABASE EMBASE [Online] DIALOG INFORMATION SERVICES, FILE 73 APFEL S.C. ET AL.: 'Neurotrophic factors in the therapy of peripheral neuropathy', XP002905394 Retrieved from Dialog, accession no. 06322661 Database accession no. 1995352901 & BAILLIERE'S CLINICAL NEUROLOGY vol. 4/3, 1995, (UK), pages 593 - 606

## Description

The present invention is related to the use of neurotrophic factors for manufacturing medical products for treating penile erectile dysfunctions.

### The Background of the Invention

The erection is predominantly a nerve-mediated vascular event. The parasympathetic pelvic nerves are of primary importance in the smooth muscle relaxation required for penile tumescence although the sympathetic hypogastric nerves may play some role as well. In man penis innervating parasympathetic nerves arise from sacral spinal cord supplying input to ganglion cells in pelvic plexus. The pelvic plexus is an association of sympathetic and parasympathetic neurons involved with eliminative and reproductive functions (Dail, 1996). Erection inducing neurons of the major pelvic ganglion (MPG) send axons via cavernous nerves to supply the blood vessels and erectile tissue of the penis (Dail, 1996; Quinlan, 1989). Recently the essential role of nitric oxide (NO), acting as a neurotransmitter in the nerves supplying the penile arteries and cavernous smooth muscle, has been well established in several mammals (Andersson, 1995). It diffuses into smooth muscle, increases the synthesis of cGMP and decreases cytosolic Ca²⁺, thus inducing relaxation (Buj-Bello, 1995). Normal penile erection in man depends on intact innervation, adequate blood supply and healthy erectile tissue (Andersson, 1995)., Therefore, interruption of neuronal pathways or disorders affecting the neuromuscular junction are likely to produce erectile dysfunction (Batra, 1991).

Despite the progress in surgical techniques neurogenic impotence is still an undesired but often encountered side-effect of radical prostatic, vesical or rectal surgery (Walsh, 1982). It is also a common symptom in autonomic and sensory neuropathies, e.g. in diabetic men (Eardley, 1991). Clinically, there is a need to discover molecules that support the survival and enhance regeneration of penile nerves.

Recently growth hormone was reported to improve regeneration of certain penile nerves in rat (Jung, 1998). The effect is suggested to be mediated by the insulin-like growth factor I (IGF-I), which is a potent neurotrophic factor for many neuronal populations in the central and peripheral nervous system (Ishii, 1993). Also the basic fibroblast growth factor (FGF-2) has been thought to play a role in the physiology of penile erection (Te, 1994). Therapeutic use of neurotrophic proteins or their small molecule mimetics as potential remedies in several diseases and insults of the nervous system have been suggested (Hefti, 1997; Skaper, 1998). However, to make a successful use of such molecules in neurogenic impotence possible, more knowledge is required about the physiological factors that regulate the maintenance and regeneration of penile neurons.

When studying GDNF. and its receptors mRNA expression in rats, the present inventors obtained results indicating that GDNF and its receptors may have a role in innervation of rat penis. Encouraged by these observations the present inventors continued their research programs and were able to found a much broader utility for neurotrophic factors. In fact a solution to some of the problems discussed above, i.e. a treatment for peripheral nerve dysfunctions of pelvic area, including but not restricted to the erectile dysfunctions was provided based on said preliminary observations.

### The Summary of the Invention

The characteristic features of the present invention are as defined in the claims.

### A Brief Description of the Drawings

**Figure 1** depicts NTN mRNA expression in the shaft of penis.
**Figure 2** depicts expression of mRNAs for NTN receptor components in MPG.
**Figure 3** depicts Northern blot analysis of NTN mRNA expression in pelvic organs of adult rat.
**Figure 4** depicts retrograde axonal transport of ¹²⁵I-NTN from the shaft of penis to the MPG and DRG.
**Figure 5** depicts NTN mRNA levels in adult rat penis after bilateral cavernous nerve axotomy (ax) or sham operation.
**Figure 6** depicts NADPH diaphorase histochemistry in penises of GFRα2^{-/-} and wild-type mice.
**Figure 7** depicts GDNF mRNA expression in the shaft of penis.
**Figure 8** depicts Northern blot analysis of GDNF mRNA expression in pelvic organs of adult rat.
**Figure 9** depicts quantification and specificity of ¹²⁵I-GDNF retrograde transport into adult rat MPG and different DRGs.
**Figure 10** depicts retrograde axonal transport of ¹²⁵I-GDNF from the shaft of penis to the MPG and DRG.
**Figure 11** depicts neurite outgrowth in mouse trigeminal ganglion at embryonal day 13 after 3 days in collagen gel.
**Figure 12** depicts expression of GFRα3 and Ret mRNAs in adult rat MPG, and of GFRα3 mRNA in the S1 DRG.
**Figure 13** depicts RNA preparation and Northern hybridization of NGF, BDNF and NT-3 mRNA expression in pelvic organs of adult rat.
**Figure 14** depicts quantification and specificity of ¹²⁵I-NGF, ¹²⁵I-BDNF, ¹²⁵I-NT-3 retrograde transport into adult rat MPG and different DRGs.

The Drawings are discussed in detail at the end of The General Description of the Invention.

**The Detailed Description of the Invention**

### References

Throughout this application various publications are referenced, many in parenthesis including the last name of the first author with the year of publication. Full citations for these publications are provided at the end of The General Description of the Invention.

### Definitions

In the present invention the terms used have the meaning they generally have in the fields of biochemistry, pharmacology, recombinant DNA technology, including transgenic animal production, but some terms are used with a somewhat deviating or broader meaning than in the normal context. Accordingly, in order to avoid uncertainty caused by terms with unclear meaning some of the terms used in this specification and in the claims are defined in more detail below.

The term "neurotrophic factors", comprises glial cell line-derived neurotrophic factor (GDNF) family-related compounds or neurotrophins (NTFs), their receptors as well as their binding substances. Also included into the definition are nucleotide sequences encoding said factors. The GDNF family-related compounds comprise neurturin (NTN), glial cell-derived neurotrophic factor (GDNF), artemin (ARTN) and/or persephin (PSPN). The neurotrophins (NTFs) comprise nerve factor (NGF), brain-derived neurotrophic factor (BDNF), neurotrophin-3 (NT-3) and/or neurotrophin 4/5 (NT-4/5).

The "neurotrophic factors" also include the conservatively substituted variants or derivatives having substantially the same effect as said neurotrophic factors on their receptors or co-receptors. The receptors or co-receptors comprises Ret tyrosine kinase and GDNF family-receptor α:s (GFRαs) and the trk protein tyrosine kinase receptors (trkA-C) as well as the receptor p75.

The "neurotrophins (NTFs)" are proteins regulating neuronal differentiation, maturation and survival. The NTFs are nerve factor (NGF), brain-derived neurotrophic factor (BDNF), neurotrophin-3 (NT-3), neurotrophin 4/5 (NT-4/5) (Davies, 1994; Sariola et al., 1994). NTFs specifically bind to and activate trk protein tyrosine kinase receptors (Barbacid, 1995). Thus, NGF activates trkA, BDNF and NT-4/5 activate trkB, and NT-3 is a ligand for trkC, but also trkA and trkB, although less efficiently. In addition to full-length, catalytic forms of the trk receptors, there are also several alternatively spliced trk isoforms. The mRNA for full-length, catalytic trkB (trkB.TK+) is alternatively spliced into two truncated isoforms (trkB.T1 and trkB.TS). Also four truncated forms are described for trkC, in addition to other isoforms with inserts in the tyrosine kinase domain.

The term "derivatives or variants" means compounds which act as the two major groups of "neurotrophic factors" of the present invention on the transmitting signals of their respective receptors. "Derivatives or variants" include polypeptides "substantially homologous" at amino acid level having a significant similarity or identity of at least 80%, preferably 85 %, most preferably more than 90 % with human forms of said neurotrophic factors.

The term "neurotrophic factors and conservatively substituted variants thereof" comprise polypeptides having the structure, properties and functions characteristic of the "neurotrophic factors" defined above. Thus, the term "neurotrophic factors and conservatively substituted variants thereof" include the neurotrophic factors mentioned above, wherein one or more amino acid residues are substituted by another amino acid residue. Also truncated, complexed or chemically substituted forms of said neurotrophic factors are included in the term. Chemically substituted forms include for example, alkylated, esterified, etherified or amidized forms with a low substitution degree, especially using small molecules, such as methyl or ethyl, as substituents, as long as the substitutions do not disturb the properties and functions of the neurotrophic factors. The truncated, complexed and/or substituted variants of said polypeptides are producible by synthetic or semisynthetic, including enzymatic and recombinant DNA techniques. The only other prerequisite is that the variants still are substantially homologous with and have the properties and/or express the functions characteristic of said neurotrophic factors.

The term "neurotrophic factors and conservatively substituted variants" covers all possible splice variants of the "neurotrophic factors" defined above. The "neurotrophic factors" can exist in different isoforms. The term "isoform" refers to the different forms of the same protein, which originate from different sources, e.g. tissues. Isoforms of a protein or polypeptide can be expressed by allelic genes present in different tissues in different species, e.g. in mammalian species of human and murine origin. In the present invention the term, includes fragments, complexes and their variants. Isoforms of neurotrophic factors can be generated by the cleavage of the proprotein. Different reactions, including different enzymatic and non-enzymatic reactions, proteolytic and non-proteolytic, are capable of creating truncated, derivatized, complexed forms of the neurotrophic factors and their conserved variants.

Preferably, all "neurotrophic factors and their conserved variants" should be recognizable when using binding substances capable of recognizing and specifically binding to natural mammalian, including human and murine neurotrophic factors or at least one specific portion of said factors. Such binding substances are for example antibodies or ligands specifically recognizing or binding to neurotrophic factors, receptors of the neurotrophic factors including GFRα:s (GFRα1-4), trk protein tyrosine kinase receptors (trkA, trkB, trkC) or other binding proteins or peptides, comprising specific portions of said GFRα:s, but above all they mean antibodies capable of specifically recognizing at least one neurotrophic factor alone or in any combination. The antibodies include both polyclonal and/or monoclonal antibodies as well as fragments or derivatives thereof. Preferably, such binding substances should recognize and bind to specific epitopes or active sites of the neurotrophic factors.

Binding substances also comprise small molecules capable of specifically binding to the "neurotrophic factors" or their respective "receptors". "Binding substances" can be produced using specific domains of said "neurotrophic factors", their isomers as well as their fragments, receptors, derivatives, variants and complexes as well as receptors with the prerequisite that they are capable of functioning in respective signaling pathway.

The "neurotrophic factors" act as antigens and are as such or as compositions capable of eliciting an antibody response specific to respective "neurotrophic factor or its conserved variant". Said antibodies are producible by conventional techniques for producing polyclonal antibodies as well as monoclonal antibodies. The methods for preparing monoclonal antibodies include hybridoma techniques. Fragments of antibodies or other binding proteins like specific binding peptides can be developed by phage display techniques and produced by recombinant DNA techniques. All methods are well known by those skilled in the art and described in laboratory handbooks.

In the present invention the term "nucleic acid sequence" means any isolated and purified nucleotide sequence or fragment therof encoding "neurotrophic factors" or "nucleic acid sequences" with substantial similarity to those preferably of mammalian, especially human origin, which nucleotide sequences are capable of encoding the neurotrophic factors of the present invention. The "nucleic acid sequences" of the present invention encode the "neurotrophic factors" of the claims and they can be used as such introducible into suitable host cells or host organisms to provide *in vitro* methods for producing neurotrophic factor proteins or for *in vivo* use for treating penile erectile dysfunctions.

The "nucleic acid sequences" of the present invention are not in their natural state but are preferably isolated and purified from their natural environment as transiently expressed mRNAs from appropriate tissues. Thereafter, the mRNAs are purified and multiplied *in vitro* in order to provide by technical means new copies, which are capable of encoding said neurotrophic factors, derivatives or variants thereof.

In the present context the term "cDNA" means a DNA sequence obtainable by reversed translation of mRNA translated from the genomic DNA sequence. Also antisense nucleotide sequences are useful in some applications of the present invention.

The term "antisense technology" means the opposite of the RNA message being in correct sense. "In sense" means that when one copy of the a strand of DNA is transcribed, the sequence of bases in the DNA is preserved, except that the sequence is exactly complementary to the original code. The mRNA, now containing instructions from DNA in its own language, is then translated into a polypeptide. If done correctly, the message is said to be in correct sense. However if an exactly complementary (i.e. antisense) RNA is present to combine with the sense RNA, the message of the sense RNA is nullified.

To make an RNA complement, an antisense message can be made from the "other strand of DNA" and introduced into a cell or it can be transcribed by the cell after an antisense gene is introduced. When the sense RNA couples or hybridizes with its antisense RNA it cannot combine with a ribosome and is inactivated. Thus the original gene is blocked by its own antisense RNA without other genes being effected. The antisense technology is included as a means of modulating and modifying the trophic support of nerves.

The term "nucleic acid sequence encoding neurotrophic factors" means nucleic acid sequences encoding glial cell line-derived neurotrophic factor (GDNF) family-related compounds or neurotrophins (NTFs), their receptors as well as their binding substances. Also included into the definition are nucleotide sequences encoding said factors. The GDNF family-related compounds comprise neurturin (NTN), glial cell-derived neurotrophic factor (GDNF), artemin (ARTN) and/or persephin (PSPN). The neurotrophins (NTFs) comprise nerve factor (NGF), brain-derived neurotrophic factor (BDNF), neurotrophin-3 (NT-3) and/or neurotrophin 4/5 (NT-4/5) as well as "substantially identical or similar nucleic acid sequences". Said "nucleotide sequences" or their complementary sequences or sequences comprising parts thereof, e.g. fragments truncated at the 3'-terminal or 5'-terminal end, as well as such nucleotide sequences containing point mutations, are especially useful as probes or primers for preparing DNA constructs for manufacturing cells or cell-lines carrying said nucleic acid sequences.

It is, however, clear for those skilled in the art that other nucleic acid sequence capable of encoding neurotrophic factors and useful for their production can be prepared. The nucleic acid sequences encoding neurotrophic factors should be capable of hybridizing under stringent condition (Sambrook, J., et al., Molecular Cloning: A Laboratory Manual., Cold Spring Harbor, NY: Cold Spring Harbor Laboratory, 1989).

The "nucleic acid sequences" of the present invention should have a "substantial similarity" with the sequences encoding neurotrophic factors defined above. "Substantial similarity" in this context means that the nucleotide sequences fulfill the prerequisites defined above and have a significant similarity, i. e. a sequence identity of at least 60%, preferably 70 % , most preferably more than 80 % with said sequences. This is also a suitable criteria for defining conservatively substituted variants. The codon degeneracy should be taken in account.

The term "nucleic acid sequences encoding neurotrophic factors" include their truncated or complexed forms as well as point mutations of said nucleic acid sequences as long as they are capable of encoding amino acid sequences having the essential structural features as well as the properties and/or functions of said neurotrophic factors. The nucleic acids are useful as such or inserted in transformation or expression vectors or hosts, said "nucleic acid sequences" being capable of encoding and expressing said "neurotrophic factors" which are recognizable by "binding substances" specifically recognizing said "neurotrophic factors" .

The term "neurotrophic factors" include in addition to the polypeptides or proteins also "nucleic acid sequences" encoding "neurotrophic factors and conservatively substituted variants thereof".

The term "composition" or "medical product" means the active ingredients in combination with at least one pharmaceutically acceptable auxiliary agent, which is compatible with the active ingredient and the route of administration. The auxiliary agents include liquid or solid carriers as well as other liquid or solid additives. The compositions can be included in a container, package or dispenser together with an injection needle and instructions for use. The nucleic acid sequences of the present invention or administrable in gene therapy by introducing the nucleotide sequences with suitable cells, vectors or by other *per se* known delivery systems.

The response elicited by the neurotrophic factors may differ. Receptor expression and retrograde transport of neurturin, GDNF, persephin artemin and/or neurotrophins in sensory nerves innervating penis suggest that these growth factors may be used to produce a medical product for treating penile erectile dysfunctions.

### The General Description of the Invention

As described in the background of the invention the erection is predominantly a nerve-mediated vascular event. The parasympathetic pelvic nerves are of primary importance in the smooth muscle relaxation required for penile tumescence although the sympathetic hypogastric nerves may play some role as well. In man penis innervating parasympathetic nerves arise from sacral spinal cord supplying input to ganglion cells in pelvic plexus. The pelvic plexus is an association of sympathetic and parasympathetic neurons involved with eliminative and reproductive functions (Dail, 1996). In the male rat, pelvic plexus is marked bilaterally by a single large ganglion, the major pelvic ganglion (MPG), which makes rat an excellent animal model in study of erection (Quinlan, 1989). Normal penile erection in man depends on intact innervation, adequate blood supply and healthy erectile tissue (Andersson, 1995). Therefore, interruption of neuronal pathways or disorders affecting the neuromuscular junction are likely to produce erectile dysfunction (Batra, 1991).

NTN is known to have survival promoting activities on e.g. sensory, sympathetic, enteric, motor, and midbrain dopaminergic neurons (Kotzbauer, 1996; Heuckeroth, 1999; Klein, 1997; Horger, 1998). Biological actions of the GDNF family members are mediated via a multicomponent receptor complex which consists of glycosylphosphatidylinositol- (GPI-) linked ligand binding a components, designated GDNF family receptors GFRα1-4, and the signal transducing receptor tyrosine kinase Ret have been demonstrated (Airaksinen, 1999).

Results of *in vitro* assays show preferential pairing of NTN with GFRα2 and GDNF with GFRα1, ARTN with GFRα3 and PSPN with GFRα4. Although there still is some promiscuity concerning the ligand specificities of the GFRα:s, recent gene ablation studies have suggested *in vivo* specificity of the interactions between GPI-linked co-receptors and GDNF family members (Airaksinen, 1999).

The striking phenotypic similarity between mice with GFRα2 or NTN gene ablation supports the concept that GFRα2 is a physiological co-receptor for NTN (Rossi, 1999; Heuckeroth, 1999). The disclosed mice have severe defects in the parasympathetic nervous system suggesting that NTN is required for the development and/or maintenance of certain post-ganglionic parasympathetic neurons.

GDNF can *in vitro* interact with multiple GFRα/Ret complexes, (Baloh, 1997; Jing, 1997; Sanicola, 1997; Suvanto, 1997) however recent gene ablation studies showing similar defects in mice deficient in GDNF or GFRα1 (Cacalano, 1998; Enomoto, 1998; Moore, 1996; Pichel, 1996; Sanchez, 1996) reveal that *in vivo* GDNF acts primarily through GFRα1. The described mice fail to develop kidneys and enteric nervous system, die in few days after birth and display similar defects both in the central and peripheral nervous system.

Peripheral nerves are known to require continuously neurotrophic factors produced in the target organ of innervation (Davies, 1994; Henderson, 1996). Neurotrophic factors have been suggested as promising drugs for treatment of injured nervous system due to their potent pharmacological effects in animal models of neurodegenerative diseases (Hefti, 1997; Skaper, 1998). Neurotrophic factors have the ability to prevent atrophy and death during development and in the adult. Although neurogenic impotence is a major problem, very little is known about neurotrophic factors important for penile erection inducing neurons. Identification of penile neurotrophic agents seems practical because their potential neuroprotective properties might have therapeutic value. GDNF was originally isolated based on its ability to promote survival and differentiation of ventral midbrain dopaminergic neurons in embryonic rat (Lin, 1993). It is a distantly related member of the transforming growth factor-b (TGF-b) superfamily and comprise GDNF family together with neurturin (NTN), (Kotzbauer, 1996) persephin (PSPN), (Milbrandt, 1998) and artemin (ARTN) (Baloh, 1998). GDNF maintains dopaminergic, noradrenergic and motor neurones of the central nervous system (Arenas, 1995; Henderson, 1995; Lin, 1993; Oppenheim, 1995; Tomac, 1995; Yan, 1995) as well as various-sub-populations of the peripheral sensory and sympathetic neurones (Buj-Bello, 1995; Ebendal, 1995; Trupp, 1995).

Furthermore, the defects in the sympathetic superior cervical ganglia in mice lacking either GDNF or GFRα3 suggest that some of the biological effects of GDNF may be mediated via GFRα3, the preferred ligand-binding receptor for ARTN (Moore et al. 1996; Sanchez et al. 1996; Baloh et al. 1998; Nishino et al. 1999).

The observation that GFRα3 mRNA is expressed mainly in the big-sized neurons of the major pelvic ganglion (MPG) conforms to the hypothesis that in adult rat ARTN may be a maintenance factor for certain pelvic adrenergic neurons. In the MPG also a subpopulation of the penis-projecting neurons expressed mRNA for GFRα3. Interestingly, the distribution of GFRα3 in the rat MPG resembles that described for neuropeptide Y (NPY) (Keast and de Groat, 1989). Whether the coreceptor GFRα3 and NPY are expressed in the same neurons remains, however, to be solved. It is further noteworthy that GFRα3 mRNA is expressed in almost half of the penile sensory neurons and preferentially in small and intermediate-sized cells. This suggests that GDNF and ARTN, the primary ligands of GFRα1 and GFRα3, may at least partially influence distinct populations of penile sensory neurons. In addition, the expression of GFRα3 mRNA in the MPG and the S1 dorsal root ganglia (DRG) hints at the possibility that also ARTN may serve as a neurotrophic factor for certain subpopulations of pelvic autonomic and sensory neurons in adult rat.

Since functional parasympathetic nerve supply is required for penile erection, the essentiality of NTN as a neurotrophic factor for penile parasympathetic neurons was studied. Rat was selected as the experimental animal because it has a tight major pelvic ganglion (MPG), which is located bilaterally to the prostate and is the main source of efferent penile NOergic nerves (Quinlan, 1989; Ding, 1993). This ganglion also contains a mixture of sympathetic and parasympathetic neurons, which innervate the internal reproductive organs, lower bowel and urinary tract.

NTN as a target derived neurotrophic factor for penile parasympathetic neurons was studied Data provided suggests that several GDNF family members may act in concert to regulate the function of penile sensory and parasympathetic neurons. To evaluate the role of GDNF and NTN for penile nerves its expression in the shaft of adult rat penis was studied by *in situ* hybridization and Northern hybridization. Immunocytochemistry was used to characterize penile cells expressing GDNF mRNA. ¹²⁵I-GDNF or ¹²⁵I-NTN was injected intracavernously and their retrograde transport were studied in sensory and parasympathetic penile nerves. To find out whether NTN mRNA synthesis in penis is under neuronal regulation its expression was studied after cavernous nerve axotomy. In GFRα2^{-/-} mice the penile innervation was studied in order to further elucidate the physiological importance of GFRα2 mediated signaling for penis projecting neurons. The results suggest a role for neurotrophins in the innervation/maintenance/regeneration of penile sensory and erection inducing neurons.

Neurturin (NTN) and other members of the glial cell line-derived neurotrophic factor (GDNF) family of neurotrophic factors, including but not restricted to artemin and persephin promotes survival of several populations of neurons in central and peripheral nervous system, including midbrain dopaminergic, sensory and motor neurons, in developing and mature rodents. NTN was identified as an endogenous trophic factor for penis projecting parasympathetic neurons. NTN mRNA was expressed in the smooth muscle of penile blood vessels and corpus cavernosum of adult rat penis, as well as in several other pelvic organs. NTN receptor components GFRα2 and Ret mRNAs were expressed ubiquitously in penile parasympathetic neurons. Retrograde transport of penis-injected ¹²⁵I-NTN in parasympathetic and sensory neurons confirmed the functionality of NTN in this system. NTN mRNA levels in penis remained unchanged after bilateral cavernous nerve axotomy. The significant decrease in nitric oxide synthase (NOS) containing nerve fibers both in the dorsal penile and cavernous nerves of GFRα2^{-/-} mice pointed to the physiological significance of GFRα2 for penile innervation.

GDNF receptor components Ret, GFRα1 and GFRα2 were shown to be expressed in penile neurons of major pelvic ganglion (MPG) and dorsal root ganglia (DRG) of adult rat. Retrograde axonal transport was inhibited by excess of unlabeled GDNF whereas excess of cytochrome c had no effect on transport suggesting that transport is mediated by binding to specific receptors located in axon terminals. *In situ* hybridization revealed productions of GDNF mRNA in the shaft of penis. GDNF mRNA expression was seen in the rows of cells at the boundary between the tunica albuginea and the erectile tissue. These cells were recognized by antibodies to vimentin and S100beta, whereas antibodies to smooth muscle alpha actin and PGP 9.5 showed no immunoreactivity. Taken together, these data suggest that NTN acts as a target-derived survival and/or neuritogenic factor for penile erection inducing postganglionic neurons. The results indicated that GDNF may act as a target derived neurotrophic factor in adult rat penis.

Characterization of the nigro-striatal dopaminotrophic activities of GDNF created considerable interest and effort to find homologous factors with similar survival promoting activities. The discovery of NTN was soon followed by the demonstration of its ability to promote survival of several neuronal populations in the peripheral and central nervous system. However, the role of NTN in pelvic organs has not been studied. It is shown here that NTN mRNA is expressed in penile smooth muscle as well as in several other pelvic organs, while penis-innervating major pelvic ganglion (MPG) neurons express mRNAs of Ret and GFRα2, and are able to specifically take up and transport NTN. Thus, NTN seems to have a role in trophic circuitry within the pelvic area. Coexpression of GFRα2 and GFRα1 in many MPG neurons raises the possibility that also other GDNF family members may regulate the function of these neurons. Similar partially overlaping expression of GFRα2 and GFRα1 mRNAs is widely seen in the central nervous system (Trupp; 1998; Sanicola, 1997; Naveilhan, 1998; Widenfalk, 1997; Golden, 1998) and in peripheral sensory and sympathetic ganglia (Yu, 1998; Bennet, 1998; Baloh, 1997). Nevertheless, the biological role of such coexpression is not well understood.

Retrograde transport from distal axon terminals to neuronal bodies is an important criterion for functionality of a neurotrophic factor for given neurons. The present invention demonstrates binding, uptake and retrograde axonal transport of NTN in penis projecting major pelvic ganglion (MPG) neurons of adult rat. This clearly shows a role for NTN as a target derived trophic factor for these neurons. A similar trophic circuit has been noted e.g. in the nigro-striatal system where intrastriatally injected GDNF is retrogradely transported by mesencephalic dopamine neurons (Tomac, 1995). The fact that ¹²⁵I-NTN, with a neurite outgrowth promoting activity comparable to that of the nonradioactive parent protein, was transported in the presence of a 100-fold excess of cytocrome c but not in the presence of a similar excess of NTN, suggests that retrograde transport was mediated by binding to specific receptors in the axon terminals. Autoradiography revealed that, in addition to the MPG, ¹²⁵I-NTN was to some extent transported to the S1 DRG, which is known to provide afferent nerves for the penis (Dailey, 1989). Low levels of radioactivity in S1 DRG are probably due to the fact that GFRα2 mRNA was expressed in only 6%, whereas GFRα1 was expressed in 57% of penis projecting neurons within S1 DRG (data not shown). Indeed, preliminary results with retrograde transport of ¹²⁵I-GDNF in penile neurons suggest that GDNF and NTN are transported mostly to distinct populations of DGR neurons. This further supports the view that *in vivo* the NTN signaling is mediated by binding to GFRα2.

The coincidence between neuron-target contact and expression of NTN mRNA in penile smooth muscle hints at the possibility that innervation might regulate production of NTN in the target. However, bilateral axotomy of the cavernous nerves did not markedly alter the amount of NTN mRNA suggesting that parasympathetic innervation does not regulate NTN expression in penile smooth muscle. This is consistent with the results of several denervation experiments of autonomic nerves in neonatal and adult animals. For example, NGF mRNA levels remain unchanged after sympathectomy both in neonatal rat heart and iris of adult rat (Shelton, 1986).

Although NTN mRNA is not increased in response to axotomy, it is possible that the NTN protein is accumulated in the target organ due to lack of retrograde transport, as reported for NGF in heart and iris after sympathectomy or in skin after axotomy of sensory nerves (Shelton, 1986; Harper, 1999). Hypothetically increased local NTN may promote reinnervation of penis as has been reported to occur after unilateral cavernous nerve axotomy, where intact axons are suggested to expand to the contralateral denervated side (Jung, 1998; Carrier, 1995). However, confirmation of this hypothesis requires additional experiments.

The recent observation that there are defects in some but not all parasympathetic neurons in GFRα2^{-/-} mice (Rossi, 1996) prompted to study whether penile NOergic innervation is altered by ablation of this gene. It was found that GFRα2-deficient mice had a significantly reduced number of NOS containing fibers both in the dorsal nerves and crura penis. Future studies will show whether decreased NOergic fiber density in penis is due to loss of neurons or reduced axonal arborization. Whatever the reason(s), the present data provide evidence of a functional role of GFRα2 mediated signaling for penile parasympathetic neurons. In view of this it could seem somewhat surprising that GFRα2^{-/-} mice are fertile (Rossi, 1999). However, the present results do not exclude subtle deficits in their reproductive performance. On the other hand, it is common that in mice with gene ablation compensatory mechanisms take over the function thought to highly depend on the deleted gene. For example, mice with targeted deletion of the neuronal NOS gene exhibit an increased expression of penile endothelial NOS (Burnett, 1996). Compensatory action of other GDNF family members is another possible explanation for partial presence of penile NOergic innervation in GFRα2^{-/-} mice. The present results suggest a compensatory role especially for GDNF, since its preferred coreceptor GFRα1 is expressed in more than half of penile MPG neurons. Support mediated by other neurotrophic factors is also a reasonable possibility.

By showing expression of NTN mRNA in penile smooth muscle and specific transport of ¹²⁵I-NTN from nerve terminals to the somata of major pelvic ganglion (MPG) neurons, a role for NTN as a trophic factor for parasympathetic penile neurons is shown. In addition, the results show that NTN mRNA expression in penis is not altered after cavernous nerve axotomy. The reduction in NOS containing nerve fibers in GFRα2^{-/-} mice points to a physiological role of GFRα2 mediated signaling for parasympathetic penile neurons in vivo.

GDNF mRNA expression in adult rat penis was confirmed by two independent methods. *In situ* hybridization showed prominent expression of GDNF mRNA in subtunical cell layer and Northern hybridization detected a transcript which size corresponds to that previously reported. In Northern hybridization GDNF mRNA was seen only in the shaft of adult rat penis, whereas other pelvic organs studied showed no hybridization. This is in contrary to wide expression of NTN mRNA in pelvic organs of adult rat. These result suggests that role of GDNF as a target derived neurotrophic factor in pelvic area may be restricted to penis only. However because neurotrophic factors are typically produced in small quantities, the possibility that GDNF mRNA is expressed in some pelvic organs in amounts under detection limit for the sensitivity cannot be excluded. This possibility is supported by wide expression of GDNF receptor components in parasympathetic and sensory neurons innervating pelvic organs.

To elucidate the function of subtunical cells expressing GDNF mRNA several antibodies were used. There was no immunoreactivity in subtunical area if sections were stained with antibodies to smooth muscle alpha actin or PGP 9.5. Antibody to smooth muscle alpha actin should recognize smooth muscle cells (Skalli, 1986) whereas antibody to PGP9.5 is an excellent panneuronal marker (Ermisch, 1995; Thompson, 1983). Absence of staining suggests that GDNF mRNA expressing cells are neither smooth muscle nor neurons. However clear signal were obtained with vimentin and S100beta antibodies. Vimentin antibody is considered as a marker for mechencymal cells, (Leader, 1987) whereas antibodies to S100beta have been reported to recognize cells of various type e.g. glial cells, chondrocytes and adipocytes. (Griffin, 1995; Haimoto, 1987) Immunoreactivity to vimentin suggests that GDNF mRNA expressing cells are of mechencymal origin. Previously Dail, et al. (1995) reported that in the rat penis the boundary between tunica albuginea and erectile tissues is marked by small cells with prominent nuclei, moderately stained for NADPH-diaphorase. (Dail, 1995) Based on the similar localization and morphology of cells observed it is suggested that these cells are same as those expressing GDNF mRNA.

The physiological role of endogenously produced GDNF in the shaft of adult rat penis remains to be shown. However expression of functional receptors in subpopulations of sensory and parasympathetic nerves favours the idea that GDNF is a target derived neurotrophic factor for these penile neurons. Another possibility is that GDNF has a local role in the shaft of penis. However this idea is less likely since *in situ* hybridization revealed no detectable Ret mRNA in the shaft of penis. Existence of other unknown signaling receptors for GDNF in penis is also a possibility, which cannot be excluced.

Retrograde transport is a common mechanism by which target derived neurotrophic factors mediate their biological effects in neuronal somata. The present findings demonstrate that biologically active rat recombinant GDNF is retrogradely transported by sensory and parasympathetic neurons following intracavernous injection. γ-counting and autoradiography revealed radioactivity both in the neurons of S1 DRG and MPG. The fact that transport of ¹²⁵I-GDNF was blocked by excess of unlabeled GDNF, but not excess of cytochrome c suggests that transport is mediated by binding to specific receptors located in the axon terminals.

GFRα1 mRNA was shown to be expressed in 54% whereas GFRα2 was expressed in 98% of penile MPG neurons. Correspondingly, GFRα1 was expressed in 57%, whereas GFRα2 was expressed only in 6% of penile sensory neurons within S1 DRG. Results concerning retrograde transport of ¹²⁵I-GDNF in penile nerves are in good correlation with receptor distribution. Transport of ¹²⁵I-GDNF in sensory nerves seems to be more effective compared to that of ¹²⁵I-NTN (more labeled cell profiles after autoradiography), whereas less radioactivity was detected in MPG after injection ¹²⁵I-GDNF. These results support the idea that transport of ¹²⁵I-GDNF in penile neurons is mediated by by GFRα1, and thus suggest that only subpopulation of penile neurons may be responsive to the trophic influences of GDNF.

Retrograde transport is significant in the pharmacological delivery of neurotrophic factors. These results suggest that intracavernous injection of GDNF in man might lead to retrograde transport and thus, exert a trophic influence on sensory and parasympathetic neurons.

The invention shows that GDNF mRNA is expressed in adult rat penis and penile sensory and parasympathetic nerve terminal are able to bind, uptake and transport ¹²⁵I-GDNF injected to the shaft of penis. Thus on the basis of the present results usage of GDNF as a therapy to neurogenic impotence seems practical.

The results suggest that NTN or GDNF can be used to produce a medical product for the treatment of impotence due to dysfunction of the cavernous nerves. The medical product is locally administrable to pelvic organs (e.g. penis), when systemically administered NTN or GDNF may enhance the regeneration and survival of pelvic plexus neurons in humans in health and disease.

NTN or GDNF may enhance regeneration and support survival of pelvic plexus neurons and so might be used to produce a medical product for treating erectile dysfunction in humans.

Receptor expression and retrograde transport of NTN or GDNF to afferent (sensory) nerves of penis suggest that these growth factors may be used to produce a medical product for treating penile erectile dysfunctions.

Consequently, a lot of evidence has accumulated based on which it can be concluded that the present invention is related to the treatment of peripheral nerve dysfunction in the pelvic area. Based on said evidence, methods for studying even in humans erectile dysfunction were developed, these methods in turn provided the production of medical products for treating penile erectile dysfunctions.

The present invention is related to the use of neurotrophic factor, which is neurturin (NTN), a glial cell-derived neurotrophic factor (GDNF), or conservatively substituted variants thereof, alone or in any combination, or nucleotide sequences encoding said neurotrophic factors for manufacturing a medical product for treating penile erectile dysfunctions. The penile erectile dysfunction is caused by acute penile trauma, penile surgery, prostatic surgery, bladder surgery or other pelvic surgery. The medical product of the present invention is preferably intracavernosally administrable. The medical product comprises in addition to the neurotrophic factor at least one optional pharmaceutically acceptable auxiliary agent which is compatible with the route of administration.

Dysfunctions, especially dysfunction in the pelvic area associated with receptor expression and or repression and retrograde transport of NTN or GDNF to afferent (sensory) nerves of penis suggested that these growth factors are useful to treat sensory neurons innervating penis and other pelvic organs. Sensory neuropathy can also be treated according to the invention by administering to the subject having the disorder a nucleic acid sequence encoding a neurotrophic factor or a portion thereof such that treament occurs, e.g. by functionally stimulating the corresponding genomic gene.

A nucleic acid sequence encoding a functional neurotrophic factor such as NTN or GDNF or conservatively substituted variants thereof may be used to prepare a medical product which is capable of being integrated into the genomic gene coding for a functional or functionally inactivatable or conditionally inactivatable neurotrophic factor and insertable into a targeting construct with a suitable selectable nucleic acid sequence acting as a marker. The targeting construct or vector is insertable and transformable into a subject suffering from the penile erectile dysfuntions.

### Example 1

### Animals

Male Wistar rats (n=16, ten to twelve weeks old), and adult GFRα2^{-/-} or wild-type mice were obtained from the animal house of the Viikki Biocenter, University of Helsinki. They were kept on a normal day and night cycle and were given free access to food an water. All efforts were made to minimize animal suffering and to reduce the number of animals used.

### Example 2

### Retrograde tracing and tissue preparation for in situ hybridization

The rats were anesthetized with a combination of midazolam (0.8 mg/kg), fentanyl citrate (0.25 mg/kg) and fluanisone (8 mg/kg), whereafter 10 µl of a 4% aqueous solution of Fluoro-Gold (FG; Fluorochrome) was injected into the corpora cavernosa. After 10 days the rats were sacrificed under anesthesia by transcardial perfusion with phosphate-buffered saline (PBS; 0.1 M sodium phosphate/0.14 M NaCI/2.6 mM KCl, pH 7.5) (50 ml) followed by fresh 4% paraformaldehyde (PFA) (200 ml) in PBS. MPG and dorsal root ganglia (DRG) from level S1 were dissected and postfixed for 1.5-2 hours at room temperature. The shafts of the penises were removed from untreated animals, fixed with 4% PFA in PBS at 4°C for 18-36 hours. After fixation all samples were rinsed in PBS and processed for paraffin sectioning at 7 *µ*m on silanized slides.

### Example 3

### In situ hybridization

In situ hybridization for sections was performed as according to Wilkinson and Green (1990) with some modifications. Briefly, sections were deparaffinized in xylene, rehydrated, fixed in 4 % (wt/vol) PFA in PBS, rinsed in PBS and treated with proteinase K (20µg/ml, Sigma) for 5-15 minutes, post-fixed with 4% (wt/vol) PFA in PBS, acetylated, dehydrated, air dried and hybridized with cRNA probe overnight at 52°C. Prehybridization (1-1.5 hours at 52°C) with hybridization mixture lacking the probe was performed. After hybridization, the sections were rinsed at high stringency for 30 minutes, treated with RNase A (Boehringer Mannheim), dehydrated and air-dried. Sections were dipped in emulsion (Kodak NTB-2), and exposed for 5 weeks. Subsequently slides were developed (Kodak D19 developer), counterstained with Harris hematoxylin and mounted in Permount (Fisher Chemical). Hybridization of adjacent sections with probes in sense orientation was used as a negative control.

### Example 4

### Image analysis and preparation of illustrations

Dark- and bright-field images were digitized using Olympus AX70 Provis microscope (Olympus Optical Co), equipped with a Photometrics SenSys CCD camera (Photometrics) and Image ProPlus 3.0 software (Media Cybernetics). Final illustrations were prepared with Adobe Photoshop 4.0 software (Adobe Systems).

### Example 5

### Quantitative analysis of receptor mRNA expression in FG-labeled neurons

Between *in situ* hybridization and emulsion autoradiography FG-labeled neurons in MPG and S1 DRG were visualized and digitized under UV-illumination. FG-labeling was compared to the *in situ* hybridization signal by using digitized images. Only brightly fluorescent neuronal profiles containing a clearly visible nucleus were counted from every fourth section throughout a ganglion. The in situ hybridization signal was considered positive if the grain density in near vicinity of a nucleus was twice as high as the background level. The expression of receptor mRNAs in FG-labeled neurons was quantified unilaterally in four rats.

### Example 6

### RNA preparation and Northern hybridization

Total RNA was isolated from frozen samples using acid guanidinium thiocyanate-phenolchloroform extraction method. (Chomczynski, 1987) (Poly-A) mRNA was purified with Oligotex mRNA Midi Kit (Qiagen). After purification (poly-A) mRNA was fractionated on a 1.2% agarose-formaldehyde gel and transferred to a nylon membrane (Magna, MSI). Following transfer the membranes were fixed by UV irradiation, prehybridized, and hybridized with ³²P-labeled cDNA probes at 65°C in 1 M NaCl, 1% sodium dodecyl sulfate, 10% dextrane sulfate and 100 µg/ml sonicated herring sperm DNA. After two high-stringency washes (0.1 x saline-sodium citrate (SSC), 0.5% sodium dodecyl sulfate at 55-60°C, 15 min) the blot was analysed by phosphoimager (Fuji BAS 1500) or at -70°C was exposed to X-ray films for 1 to 7 days at -70°C. To compare the relative levels of mRNA in different lanes, the filter was rehybridized with glyceraldehyde 3-phosphate dehydrogenase (GAPDH) probe.

### Example 7

### cDNA and cRNA probes

For *in* s*itu* hybridization single-stranded antisense and sense cRNA probes were generated and labeled with ³⁵S-UTP (Amersham) using appropriate RNA polymerases (SP6, T7, Promega). DNA templates were rat GFRα1 (nucleotides 294-1039 of U59486) rat GFRα2 (nucleotides 85-257 of AF003825) mouse NTN (nucleotides 349-936 of U78109), a gift from Dr. Carlos F. Ibáñez, and rat Ret (nucleotides 21-200 of U22514). For Northern hybridization cDNA probes were labeled with ³²P-dCTP using the Prime-a-Gene Labeling System (Promega). DNA templates for preparing ³²P-dCTP labeled cDNA probes were mouse NTN (same nucleotides as for in situ hybridization), and rat GAPDH (nucleotides 137-949 of X02231). Rat GFRα2, Ret, and GAPDH cDNAs were cloned by PCR. The identity of cloned fragments was verified by direct sequencing with a Pharmacia A.L.F. automatic DNA sequencer.

### Example 8

### Preparation and bioactivity test of ¹²⁵I-labeled NTN (¹²⁵I-NTN)

Human recombinant NTN produced in *E. coli* (PeproTech) was radioiodinated by the lactoperoxidase method (Marchalonis, 1969). The following ingredients were mixed and kept at room temperature for 18 minutes in final volume of 50 *µ*l: 1 mCi Na¹²⁵I (1Ci=37GBq), 10 µg NTN protein, 0.5 U lactoperoxidase, 2 *µ*l 0.03 % H₂O₂ and 0.1 M sodium phosphate buffer, pH 6.0. After the first 9 minutes of incubation, an additional 2 *µ*l of H₂O₂ was added. The reaction was terminated by adding 3 volumes of buffer containing 0.42 M NaCl, 0.1 M NaI and 0.1 M sodium phosphate buffer, pH 7.5, and 22 *µ*l of 2.5% BSA in 0.1 M sodium phosphate buffer, pH 7.5. Specific activities and labeling efficiencies were calculated after trichloroacetic acid precipitation of an aliquot of the reaction mixture. Free iodine was separated and ¹²⁵I-NTN was concentrated by using NanoSpin Plus columns (4000 MWCO, Gelman Sciences). Radioactivity was measured with a 1271 Riagamma counter (Wallac). ¹²⁵I-NTN had a specific activity of 44.7 µCi/µg. Labeled NTN was bioassayed for stimulation of neurite outgrowth from embryonal day 13.5 mouse trigeminal ganglia explanted in collagen matrix and stained with neurofilament antibodies (Arumae, 1993; Ebendahl, 1989).

### Example 9

### ¹²⁵I-NTN injections and retrograde transport

The rats were anesthetized as described in Example 2, whereafter solutions containing 200 ng of ¹²⁵I-NTN alone or combined with 20 µg of unlabeled NTN or cytochome c in a volume of 5 µl were injected into both corpora cavernosa using a 10 µl Hamilton syringe (1701N). Rats were killed 24 h later under deep anesthesia by transcardial perfusion with PBS (50 ml) followed by 4% PFA in PBS (250 ml). After perfusion MPG and DRG (levels L4-S2) were dissected and post-fixed for 1.5-2 h. Radioactivity of ganglia was measured by γ-counting. After fixation cryostat sections (10 µm) were prepared, dehydrated, dipped in emulsion, and exposed for 2-3 weeks. Then slides were developed, counterstained with hematoxylin and mounted.

### Example 10

### Cavernous nerve axotomy

Adult rats were anesthetized with a combination of ketamin (105 mg/kg) and xylazin (6 mg/kg). Cavernous nerves were exposed and cut bilaterally just distal to the MPG. To minimize religation of the nerve segments, both transected ends were deflected. In the sham operated rats the cavernous nerves were identified but not cut. Rats were sacrificed at 1, 3, 7 and 14 days after axotomy. The shafts of the penises were dissected, frozen in liquid nitrogen and stored at -70°C. mRNA was isolated and Northern blot were prepared as described in Example 6.

### Example 11

### NADPH diaphorase staining

The shafts of the mice penises were fixed for 5 hours in cold 4% PFA in PBS. Tissues were cryoprotected overnight in cold 30% sucrose in PBS. They were embedded in Tissue-Tek O.C.T. compound (Sakura), frozen on dry ice and stored at -70°C. Cryostat sections (10 µm) were prepared and adhered on Super Frost Plus slides (Menzel-Gläser). After brief air-drying they were incubated with 0.1 mM NADPH, 0.2 mM nitroblue tetrazolium, and 0.2% Triton X-100 in 0.1 M sodium phosphate buffer, pH 8.0, for 90 minutes at room temperature. The reaction was terminated by washing with water. The sections were coverslipped with Mowiol-based mounting medium. The staining pattern was assessed by counting unilaterally the number of NADPH-positive nerve fibers present in 3 random fields (400x) of corpus cavernosum and in the entire dorsal nerve.

### Example 12

### cDNA and cRNA probes

For *in situ* hybridization single-stranded antisense and sense cRNA probes were generated and labeled with ³⁵S-UTP (Amersham) using appropriate RNA polymerases (SP6, T7, Promega).DNA template used was rat GDNF (nucleotides 52-688 of L15305). For northern hybridization cDNA probes were labeled with ³²P-dCTP using Prime-a-Gene Labeling System (Promega). DNA templates used for preparing ³²P-dCTP labeled cDNA probes were rat GDNF (same nucleotides as for in situ hybridisation), and rat GAPDH (nucleotides 137-949 of X02231).

### Example 13

### Preparation and bioactivity of ¹²⁵I-Labeled GDNF (¹²⁵I-GDNF)

Rat recombinant GDNF produced by expressing the gene in rbaculovirus-infected insect cells was radioiodinated with Bolton & Hunter reagent. (Bolton, 1973) GDNF protein (10 µg) in 20 µl of 0.1M borate buffer, pH 8.5 was placed in the dried 2mCi (1Ci=37GBq) of ¹²⁵I-labeled Bolton-Hunter reagent, and the reaction mixture was incubated at 4°C for 15 minutes. Reaction was stopped by adding 0.5 ml solution containing 0.2M glycine in 0.1M borate buffer, pH 8.5. Free iodine and glycine conjugate were separated and ¹²⁵I-GDNF was concentrated by using NanoSpin Plus columns (4000 MWCO, Gelman Sciences). Radioactivity was measured with a Wallac 1271 Riagamma gamma counter (Wallac). ¹²⁵I-GDNF had a specific activity of 61.3 µCi/µg. Labeled GDNF was bioassayed for stimulation of neurite outgrowth from explanted E13-E13.5 mouse trigeminal ganglia in collagen matrix as described.(Arumäe, 1993; Ebendal, 1989)

### Example 14

### ¹²⁵I-GDNF injections and emulsion autoradiography

The rats were anaesthetized as described in Example 2 and solutions containing 200ng of ¹²⁵I-GDNF alone or combined with 20µg of unlabeled GDNF or cytochome c in a volume of 5µl was injected into the both corpora of the penis using a Hamilton syringe equipped with 26G needle. Animals were killed 24 hours later by transcardial perfusion under deep anaesthesia with PBS (50 ml) followed by 4% PFA in PBS (250 ml). After perfusion major pelvic ganglia and dorsal root ganglia (levels L4-S2) were collected and post-fixed for 1.5-2 h. During post-fix period ganglia were used for γ-counting. After fixation cryostat sections (10 *µ*m) were prepared, dehydrated, dipped in emulsion (Kodak NTB-2), and exposed for 2-3 weeks. Then slides were developed (Kodak D19 developer), counterstained with Harris hematoxylin and mounted in Permount (Fischer Chemical).

### Example 15

### Immunocytochemistry

The shafts of the rat penises were immersion fixed for 17-18 hours in a cold freshly prepared 4% (wt/vol) PFA in PBS. After fixation samples were rinsed in PBS and processed for paraffin sectioning at 10 µm on Super Frost Plus slides (Menzel-Gläser). Sections were deparaffinized in xylene, rehydrated, and incubated for 1 hour at room temperature in PBS with 4% BSA and 0.4% Triton-X 100, then for 18 hours with primary polyclonal antibodies against S100beta (1:5000, Swant), PGP 9.5 (1:2000, Affinity) or mouse monoclonal antibodies against Vimentin (1:20, Amersham) or smooth muscle alpha actin (1:200, Progen). Staining was visualized with an avidin-biotin-peroxidase system (Vector Laboratories) with diaminobenzidine as the chromogen. Finally sections were dehydrated and mounted in Permount (Fischer Chemical).

### Example 16

### cDNA and cRNA probes

For *in situ* hybridization single-stranded antisense and sense cRNA probes were generated and labeled with ³⁵S-UTP (Amersham Pharmacia Biotech) using appropriate RNA polymerases (SP6, T7, Promega, Madison, WI, USA). The DNA templates was GFRα3 (nucleotides 81-489 of AF184920).

### Example 17

### cDNA and cRNA probes

For *in situ* hybridization single-stranded antisense ' and sense cRNA probes were generated and labeled with ³⁵S-UTP (Amersham) using appropriate RNA polymerases (SP6, T7, Promega). The probes for rat NGF, BDNF, NT-3, trkA, trkB.TK+, trkC.TK+ and p75 have been described previously.(Arumae et al., 1993; Pirvola et al., 1992: Pirvola et al., 1994; Ylikoski et al., 1993; Hiltunen et al., 1996) Trk.TK+ probe recognizes all four TK domain-containing isoforms and corresponds to rat trkC cDNA sequence nucleotides 2308-2552. (Merlio et al., 1992) BDNF fragment traverses nucleotides 517-882 of rat BDNF cDNA sequence (Maisonpierre et al., 1991) and localizes in the mature BDNF mRNA except for the first two nucleotides which belong to the proBDNF mRNA. DNA templates for preparing ³²P-dCTP labeled cDNA probes were same as for in situ hybridization, and for rat GAPDH (nucleotides 137-949 of X02231).

### Example 18

### Preparation and bioactivity of ¹²⁵I-Labeled NTFs (¹²⁵I-NTFs)

Recombinant proteins were radioiodinated with lactoperoxidase method (Marchalonis, 1969). Specific activities and labeling efficiencies were calculated after trichloroacetic acid precipitation of an aliquot of the reaction mixture. Free iodine was separated and ¹²⁵I-NTFs was concentrated by using NanoSpin Plus columns (4000 MWCO, Gelman Sciences). Radioactivity was measured with a 1271 Riagramma counter (Wallac). ¹²⁵I-NTNs (NGF, NT-3 and BDNF) had a specific activity of 30-100 µCi/µg. Labeled NTFs were bioassayed for stimulation of neurite outgrowth from embryonal day 9-10 chick DRGs explanted in collagen matrix as described (Arumäe, 1993; Ebendal, 1989).

### Example 19

### ¹²⁵I-NTFs injections and retrograde transport

The rats were anaesthetized as described in Example 2, whereafter solutions containing 200ng of ¹²⁵I-NTFs (NGF, NT-3 or BDNF) alone or combined with 20µg of unlabeled NTFs or cytochome c in a volume of 5µl was injected into the both corpora cavernosa using a Hamilton syringe (1701N). Rats were killed 24 h later under deep anaesthesia by transcardial perfusion with PBS (50 ml) followed by 4% PFA in PBS (250 ml). After perfusion major pelvic ganglia and dorsal root ganglia (levels L4-S2) were dissected and post-fixed for 1.5-2 h. Radioactivity of ganglia was measured by γ-counting. After fixation cryostat sections (10 *µ*m) were prepared, dehydrated, dipped in emulsion, and exposed for 2-3 weeks. Then slides were developed, counterstained with hematoxylin and mounted.

### Results 1

### Expression of NTN in the penis, and of GFRα2, GFRα1, and Ret in the MPG and DRG of adult rat

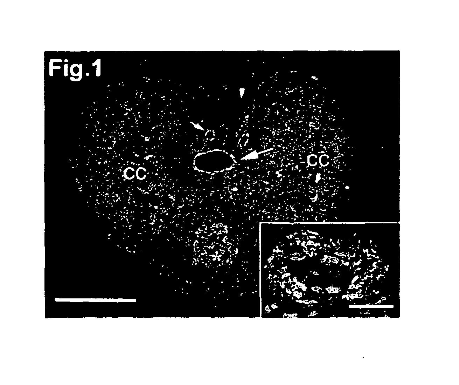

The results presented in Fig. 1 show that NTN mRNA is widely expressed in the smooth muscle of penile blood vessels and corpus cavernosum. Hybridization was seen both in arteries and veins and to some extent also in the epithelium of the urethra. *In situ* hybridization showed no detectable expression of Ret or GFRα2 in the shaft of penis and only weak and diffuse GFRα1 expression was seen in corpus cavernosum. However, Northern hybridization revealed weak expression of all receptor mRNAs in the shaft of penis (data not shown).

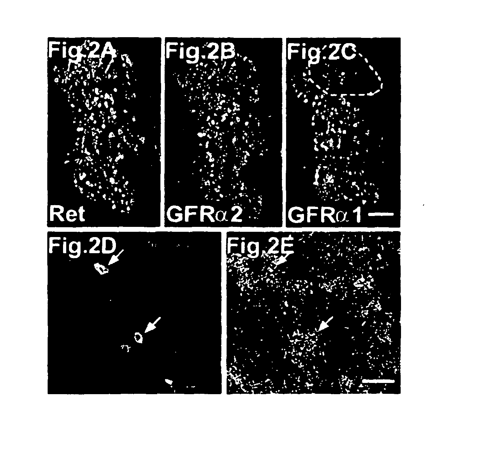

Ret and GFRα2 mRNAs were expressed in most neurons of the MPG (Figs. 2A and B). In penis projecting, i.e. FG-positive, MPG neurons expression was seen in all and 98%, respectively (Figs. 2D and E). GFRα1 mRNA was expressed in 54% of penile MPG neurons. In the area of FG-labeled neurons there were small neurons which did not express GFRα1 mRNA. Otherwise GFRα1 seemed to be expressed evenly throughout the MPG (Fig. 2C). Within S1 DRG of adult rat Ret, GFRα1 and GFRα2 mRNAs were expressed in 62%, 57% and 6% of penile neurons, respectively (data not shown). Ret mRNA expression within S1 DRG was seen in cells of all size, whereas GFRα1 mRNA was expressed preferentially in intermediate and large-sized cells, and GFRα2 mRNA in small and intermediate-sized cells. There was no detectable expression of NTN mRNA in the MPG or S1 DRG.

### Results 2

### Expression of NTN in other pelvic organs

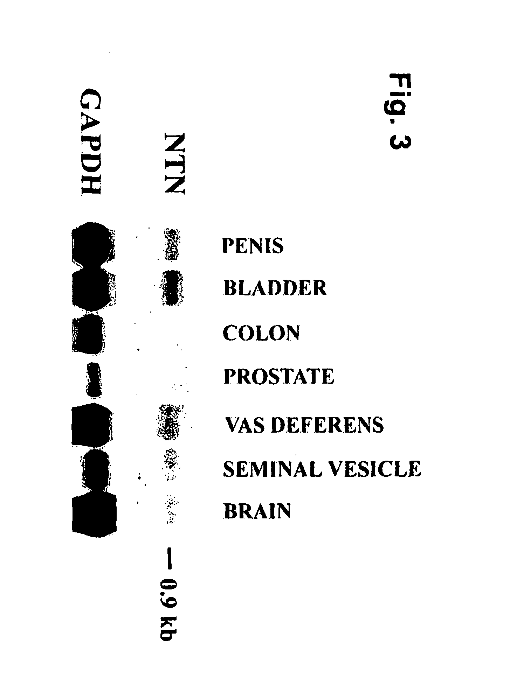

A single NTN transcript of 0.9 kb was detected in all pelvic organs studied (Fig. 3). Highest expression was seen in the penis, urinary bladder and vas deferens, whereas the signal in colon, prostate and seminal vesicles was weaker.

### Results 3

### Retrograde transport of ¹²⁵I-NTN in penis projecting neurons

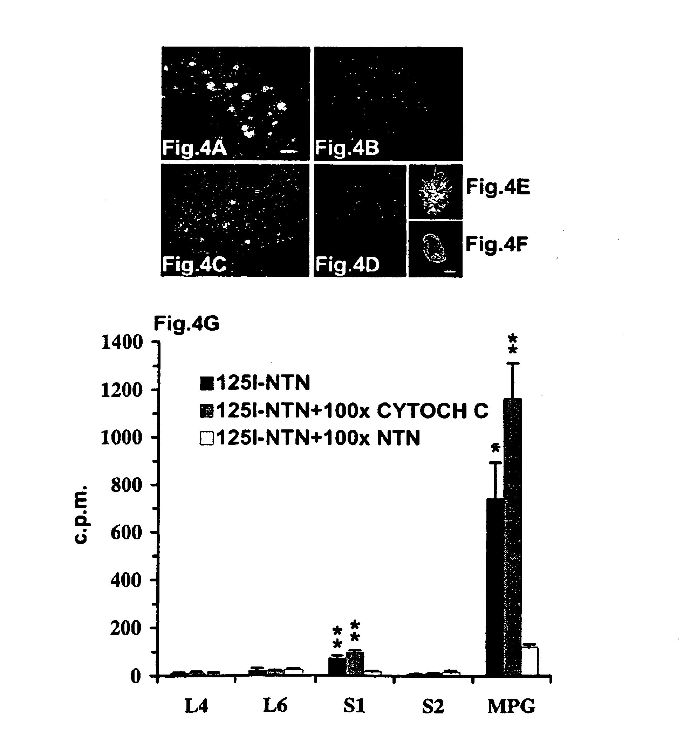

¹²⁵I-NTN (20 ng/ml) induced strong neurite outgrowth in mouse embryonic trigeminal ganglia (Fig. 4E), while there were no neurites in control ganglia cultured in the absence of neurotrophic factors (Fig. 4F). ¹²⁵I-NTN and nonradioactive NTN exhibited similar neuritogenic efficacy at all concentrations tested (5-20 ng/ml, data not shown). 24 hrs after injection of 200 ng of ¹²⁵I-NTN into both corpora cavernosa, autoradiography revealed labeling in MPG and S1 DRG (Figs. 4A and C). Although the resolution of autoradiography is limited the clustered silver grains suggest that label is present exclusively in nerve cell bodies. To show the specificity of the transport the same amount of ¹²⁵I-NTN was injected together with excess unlabeled NTN. This blocked the retrograde transport because radioactivity in MPG and DRG became hardly if at all detectable (Figs. 4B, D and G). Fig. 4G shows that after injection of ¹²⁵I-NTN significant amounts of radioactivity were detected only in ganglia innervating the penis, i.e. the MPG and S1 DRG. To exclude the blocking effect of excess protein, ¹²⁵I-NTN was also injected with excess cytochrome c. This increased the retrograde transport of ¹²⁵I-NTN possibly indicating that excess cytochrome c can inhibit unspecific binding of ¹²⁵I-NTN to syringe and tissues.

### Results 4

### NTN mRNA expression in the penis after bilateral cavernous nerve axotomy

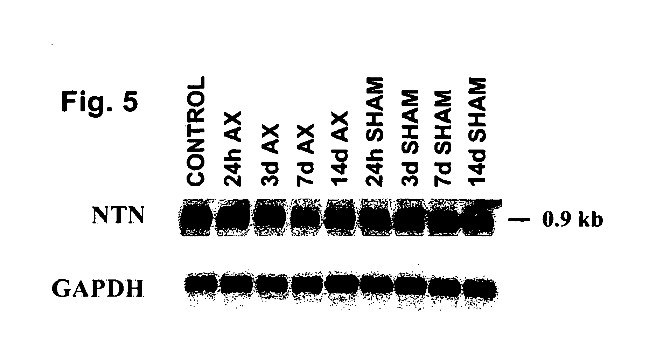

Fig. 5 shows that NTN mRNA expression in penis remains relatively unchanged after bilateral cavernous nerve axotomy.

### Results 5

### Loss of NADPH diaphorase staining in penile nerves of GFRα2^{-/-} mice

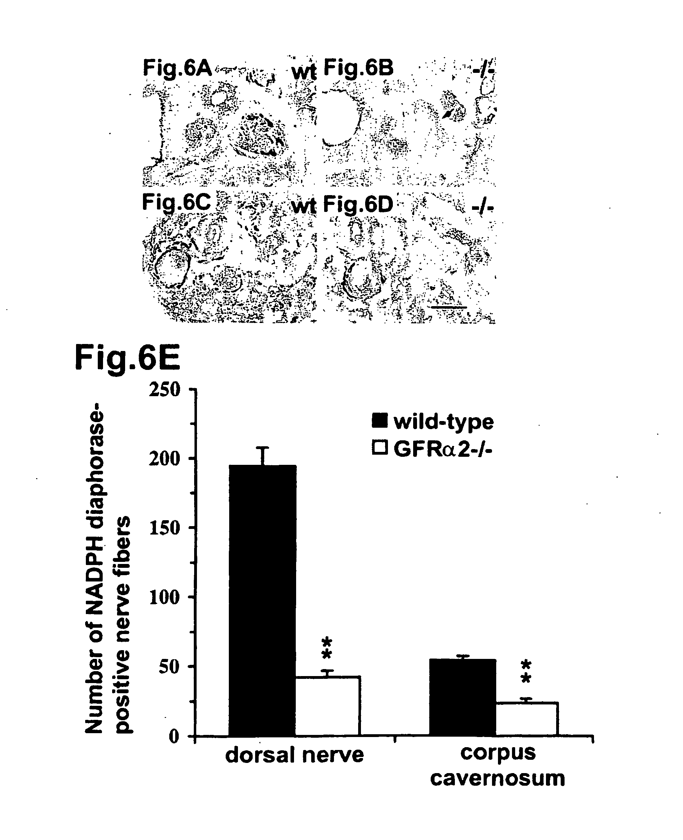

On histochemical examination of the proximal part of the shaft and crura penis from wild-type and GFRα2^{-/-} mice, a clearly distinct pattern of NADPH-positive nerve fibers was noted. GFRα2^{-/-} mice had very few NADPH-positive fibers in the dorsal nerves, while the fiber density in wild-type mice was similar to that reported in rats (Jung, 1998) (Figs. 6A, B and E). There was no clear difference in the staining pattern of nerves supplying cavernosal vessels in the crura (Figs. 6C and D). However, in the crura NADPH-positive fibers were significantly reduced outside the perivascular area. (Figs. 6C, D and E).

### Results 6

### Expression of GDNF mRNA in the shaft of adult rat penis

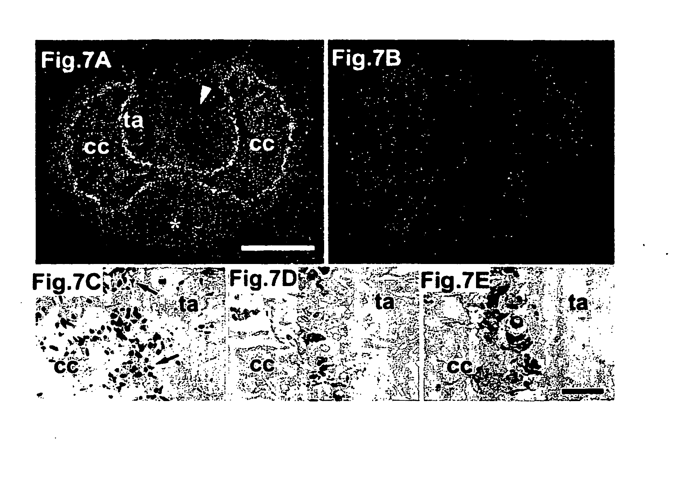

Autoradiogram of tissue section obtained after hybridization with ³⁵S-labeled GDNF riboprobe is seen in Fig. 7A. Strong signal is seen in the cell layer located in the boundary between the tunica albuginea and the erectile tissue (Fig. 7A, C), whereas there were no detectable signal in sections hybridized with sense probe (Fig. 7B). GDNF mRNA expressing cells have prominent nuclei as revealed by hematoxylin staining (Fig. 7C). Staining of adjacent sections with antibodies to vimentin or S100beta revealed immunoreactivity in these cells (Fig. 7D, E). Whereas staining with antibodies to PGP 9.5 and smooth muscle alpha actin showed no immunoreactivity in these cells (data not shown).

### Results 7

### Expression of GDNF mRNA in other pelvic organs

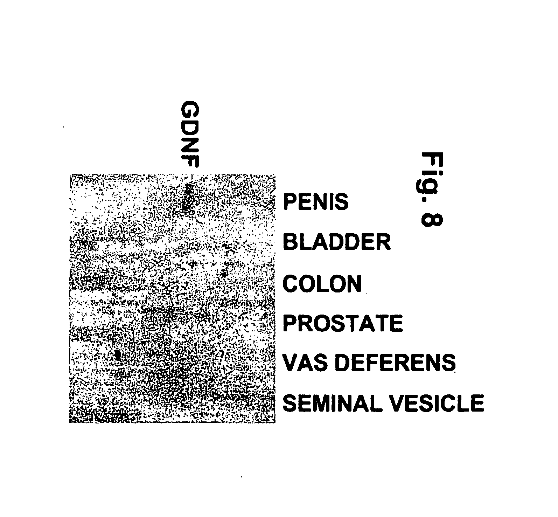

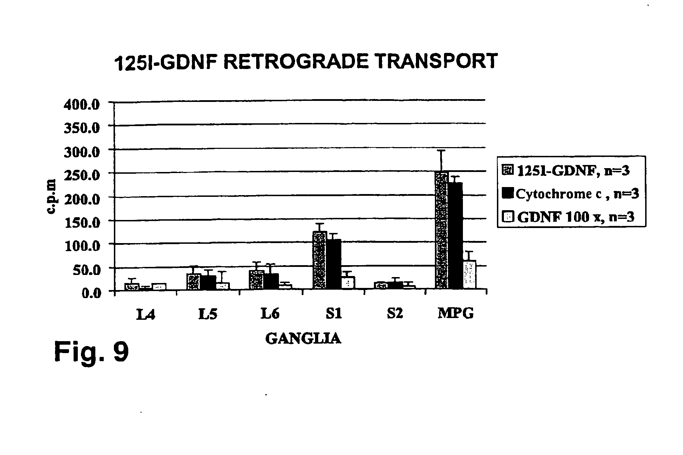

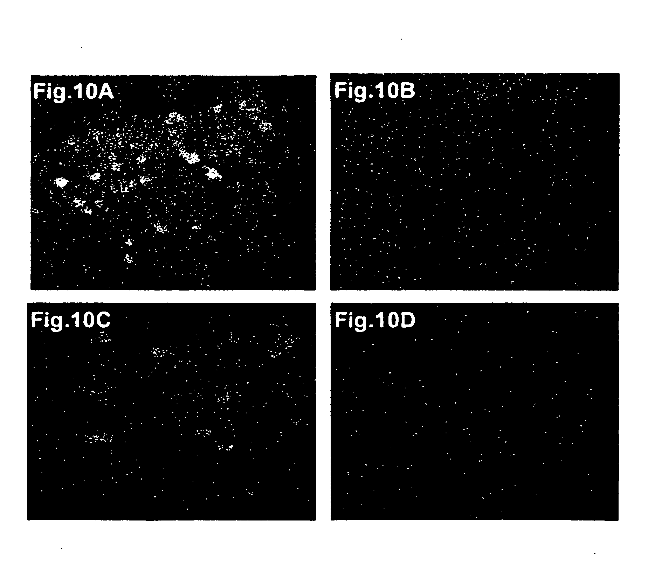

A single GDNF transcript of 4.4 kb was seen in the penis (Fig. 8). Whereas there were no detectable signal in urinary bladder, vas deferens, colon, prostate or seminal vesicles. Retrograde transport of ¹²⁵I-GDNF in penis projecting neurons 24 hours after injection of 200 ng of ¹²⁵I-GDNF into both corpora cavernosa, autoradiography revealed labeling in MPG and S1 DRG (Fig. 10 A, C). In sensory ganglia labeling was seen in a higher proportion in intermediate and large-sized neurons. Despite the limited resolution of autoradiography, the clustered silver grains suggest that label is present exclusively in nerve cell bodies. To show the specificity of the transport the same amount of ¹²⁵I-GDNF was injected together with excess unlabeled GDNF. This blocked the retrograde transport to MPG and DRG (Fig. 10B and D and Fig. 9). Fig. 9 shows that after intracavernous injection of ¹²⁵I-GDNF significant amounts of radioactivity were detected only in neurons innervating the penis, i.e. the MPG and S1 DRG. To exclude the blocking effect of excess protein, ¹²⁵I-GDNF was also injected with excess cytochrome c. This did not affect to retrograde transport of ¹²⁵I-GDNF.

### Results 8

### Biological activity of radiolabeled GDNF

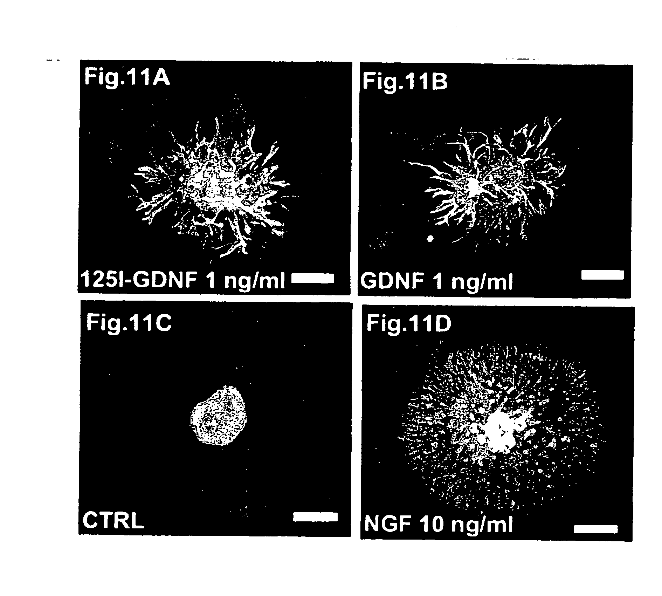

¹²⁵I-GDNF and parent GDNF (1 ng/ml) induced comparable neurite outgrowth in mouse embryonic trigeminal ganglia (Fig. 11A and B), while there were no neurites in control ganglia cultured in the absence of GDNF (Fig. 11C). Neurite outgrowth promoting activity was similar between ¹²⁵I-GDNF and nonradioactive GDNF at all concentrations tested (1-20 ng/ml, data not shown).

### Results 9

### Expression of NT-3, NGF and BDNF mRNAs in the shaft of adult rat penis

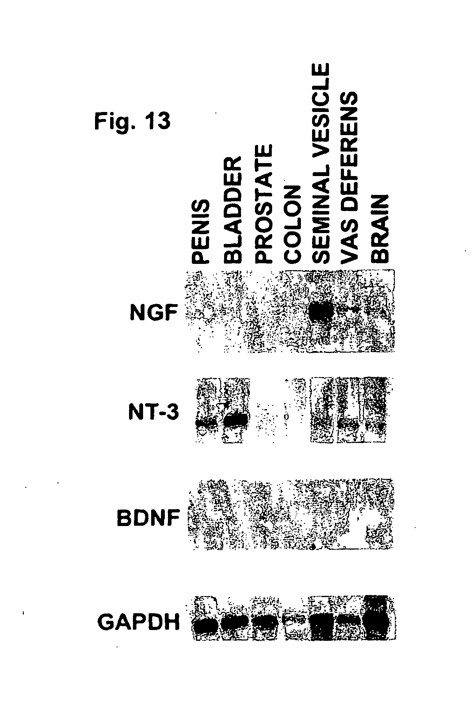

NT-3 and NGF mRNA expression in the adult rat penis was seen in the shaft of adult rat penis by in situ hybridization (data not shown) and Northern blotting (Fig. 13).

### Results 10

### Retrograde transport of NGF, BDNF and NT-3 in penile neurons

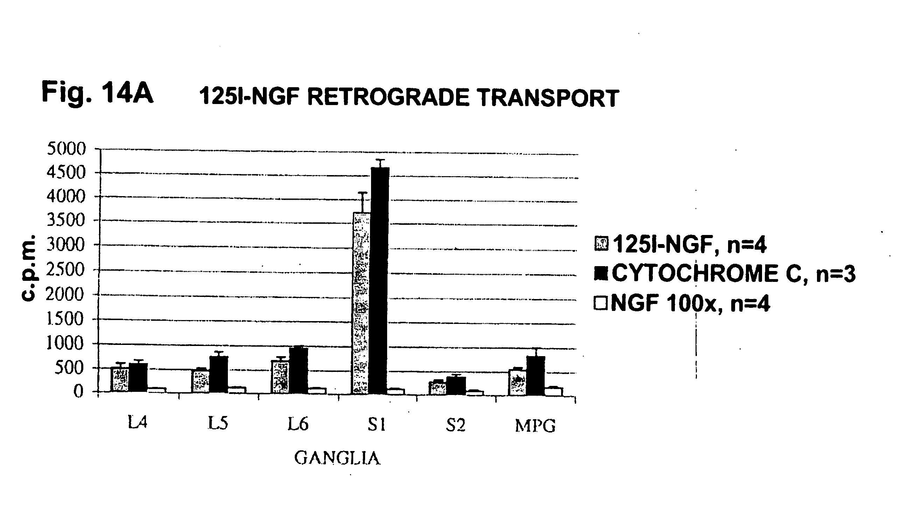

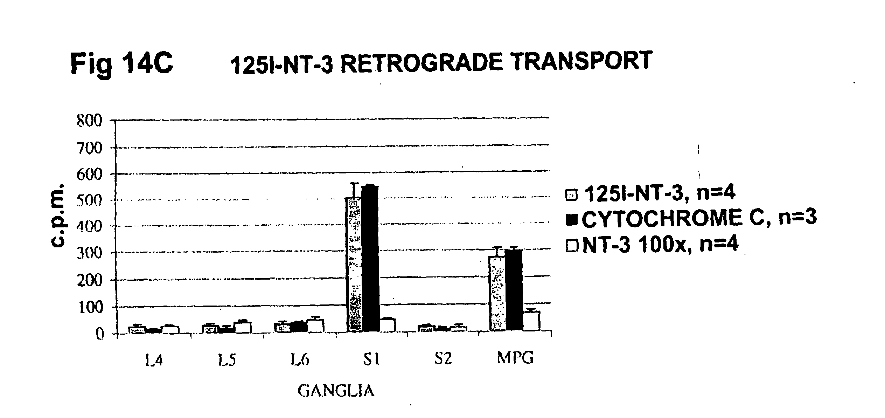

All proteins studied were transported to sensory ganglia innervating adult rat penis after injection to shaft of penis, indicating existence of functionals receptors. Only NT-3 was transported to major pelvic ganglion. (Fig. 14A-C).

### Results 11

### Expression of GFRα3 mRNA in the MPG and the S1 DRG

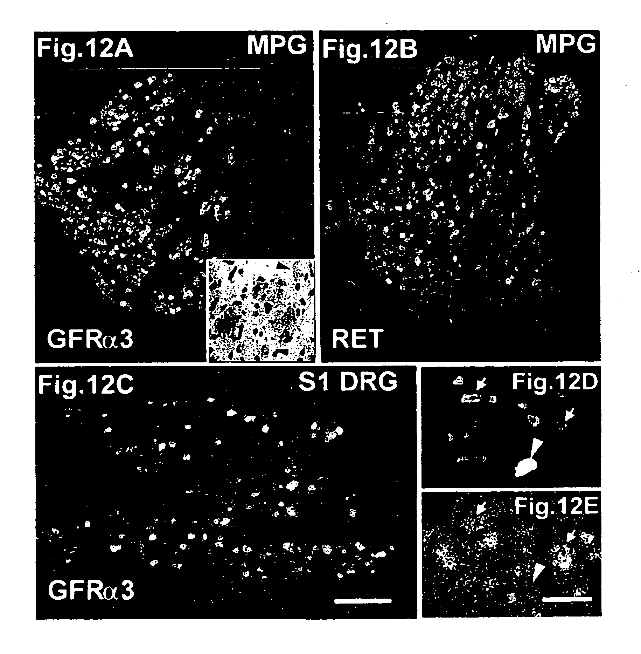

In the MPG of adult rat, GFRα3 mRNA was expressed in most of the big-sized, i.e. non-penis projecting short adrenergic neurons (Dail 1996), but only in few of the small-sized, i.e. cholinergic neurons (Fig. 12A), whereas Ret mRNA expression was seen throughout the ganglion (Fig. 12B). Accordingly, GFRα3 mRNA was expressed in 5.3 ±2.6 % of the penis-projecting, that is FG-positive, MPG neurons (mean±S.D., FG-labeled neurons were quantified unilaterally in three rats, n=414). Within the S1 DRG, expression of GFRα3 mRNA was seen preferentially in small and intermediate-sized cells (Fig. 12C) and was detected in 45.6±6.3 % of the penis-projecting neurons (Fig. 12D,E) (mean±S.D., FG-labeled neurons were quantified unilaterally in three rats, n=505).

### The Detailed Description of the Drawings

**Figure 1.** NTN mRNA expression in the shaft of penis. Hybridization can be seen in dorsal arteries (small arrow), dorsal vein (big arrow) and in blood vessels and smooth muscle of corpus cavernosum (cc), as illustrated by darkfield images of autoradiograms. Asterisk denotes urethra, arrow head points to dorsal nerve and insert represents hybridization in intracavernosal blood vessel. [Scale bars: 1 mm; insert, 20 *µ*m.]
**Figure 2.** Expression of mRNAs for NTN receptor components in MPG. (A-C) Darkfield images of adjacent sections show almost ubiquitous signals for Ret, GFRα2 and GFRα1 mRNAs. (C) Stippled circle indicates an area rich in penis projecting neurons. In this area GFRα1 signal is weaker. (D an E) Arrows point out MPG neurons colabeled with FG (D) and GFRα2 probe (E). [Scale bars: A-C, 150µm; D and E, 50µm.]
**Figure 3.** Northern blot analysis of NTN mRNA expression in pelvic organs of adult rat. A ³²P-labeled NTN probe was hybridized to the blot with mRNA from the indicated tissues (3 *µ*g/lane). The brain served as a positive control. Hybridization of the GAPDH probe denotes loading.
**Figure 4.** Retrograde axonal transport of ¹²⁵I-NTN from the shaft of penis to the MPG and DRG. (A and C) Autoradiography shows punctate signal over neuronal cell bodies in MPG (A) and S1 DRG (C) 24 hours after injection of ¹²⁵I-NTN (400 ng) into the corpora cavernosa. (B and D) Excess of unlabeled NTN abolished retrograde transport of ¹²⁵I-NTN to MPG (B) and 51 DRG (D). (E and F) Neurite outgrowth in mouse trigeminal ganglion at embryonal day 13 after 3 days in collagen gel in the presence of 20 ng/ml of ¹²⁵I-NTN (E) and in its absence (F). Neurites were visualized by antineurofilament staining. (G) Quantification and specificity of ¹²⁵I-NTN retrograde transport into adult rat MPG and different DRGs. ¹²⁵I-NTN was injected into the penis alone or with 100-fold excess of unlabeled NTN or cytochrome c. Results are expressed as mean ± SEM of 4 animals in every group. *, P<0.05; **, P<0.01 (ANOVA using Scheffe's correction). [Scale bars: A-D, 100 µm; E and F, 250 µm.]
**Figure 5.** NTN mRNA levels in adult rat penis after bilateral cavernous nerve axotomy (ax) or sham operation. Northern hybridization shows that NTN mRNA expression is not significantly affected. Control represents mRNA from untreated rats. Every lane corresponds to pooled mRNA from five rats. Equal loading is verified with GAPDH hybridization.
**Figure 6.** NADPH diaphorase histochemistry in penises of GFRα2^{-/-} and wild-type mice. Cross sections of the proximal part of the shaft of the penis of wild-type (A) and GFRα2^{-/-} mice (B). Staining of dorsal penile nerves (arrow) is dramatically diminished in GFRα2^{-/-} mice. (C and D) In the crura penis staining of nerve fibers is clearly reduced although perivascular staining seems to be less affected. (E) Quantification of NADPH diaphorase-positive fibers in dorsal nerves and in crural corpus cavernosum. Results are expressed as mean ± SEM of 4 wild-type and 4 GFRα2^{-/-} mice. **, P<0.01 (Two-tailed Student's t-test assuming unequal variances). [Scale bars: A-D, 100 *µ*m].
**Figure 7.** GDNF mRNA expression in the shaft of penis (A, C). Hybridization can be seen in the boundary between tunica albuginea and corpus cavernosum. (C) Higher magnification shows that subtunical cells expressing GDNF mRNA have prominent nuclei as revealed by hematoxylin staining. (B) Darkfield image of section incubated with sense probe shows no hybridization. (D and E) Adjacent sections stained with antibodies to vimentin (D) or S100beta (E) revealed immunoreactivity in subtunical cells. Asterisk denotes urethra, arrow head points to dorsal nerve. cc, corpus cavernosum; ta, tunica albuginea. [Scale bars: A and B, 1 mm; C-E, 50 µm.]
Figure 8. Northern blot analysis of GDNF mRNA expression in pelvic organs of adult rat. A ³²P-labeled GDNF probe was hybridized to the blot with mRNA from the indicated tissues (3 *µ*g/lane).
**Figure 9.** Quantification and specificity of ¹²⁵I-GDNF retrograde transport into adult rat MPG and different DRGs. ¹²⁵I-GDNF was injected into the penis alone or with 100-fold excess of unlabeled NTN or cytochrome c. Results are expressed as mean ± SEM of 4 animals in every group. *, P < 0.05; **, P < 0.01 (ANOVA using Scheffe's correction).
**Figure 10.** Retrograde axonal transport of ¹²⁵I-GDNF from the shaft of penis to the MPG and DRG. (A and C) Autoradiography shows punctate signal over neuronal cell bodies in MPG (A) and S1 DRG (C) 24 hours after injection of ¹²⁵I-GDNF (400 ng) into the corpora cavernosa. (B and D) Excess of unlabeled GDNF abolished retrograde transport of ¹²⁵I-GDNF to MPG (B) and S1 DRG (D). [Scale bars: A-D, 100 *µ*m.]
Figure 11. (A and B) Neurite outgrowth in mouse trigeminal ganglion at embryonal day 13 after 3 days in collagen gel in the presence of 1 ng/ml of ¹²⁵I-GDNF (A), GDNF (B) or in the absence of neurotrophic factors (C). Neurites were visualized by antineurofilament staining. [Scale bars: A-C, 500 µm.]
**Figure 12.** Expression of GFRα3 and Ret mRNAs in adult rat MPG, and of GFRα3 mRNA in the S1 DRG. (A,B) Darkfield images of adjacent sections show prominent signals for GFRα3 and Ret in the MPG. The GFRα3 signal is seen mainly over big-sized neurons (A), whereas the Ret signal is seen throughout the ganglion (B). Insert in A represents brightfield image of the MPG section hybridized with the GFRα3 probe. Arrowheads point to small-sized neurons which do not express GFRα3 mRNA. (C) Darkfield image showing GFRα3 signal over neurons in the S1 DRG. (D,E) Small arrows indicate S1 DRG neurons colabeled with FG (D) and the GFRα3 probe (E). Arrow head points to a FG-labeled neuron (D), which does not express GFRα3 mRNA (E). [Scale bars, (A-C) 250 *µ*m; (D,E) 50 µm.]
**Figure 13.** Nothern blot analysis of NGF, BDNF and NT-3 mRNA expression in pelvic organs of adult rat. ³²P-labeled probes were hybridized to the blots with mRNA from indicated tissues. (3 µg/lane). The braon serves as positive control. Hybridization of GAPDH probe denotes loading.

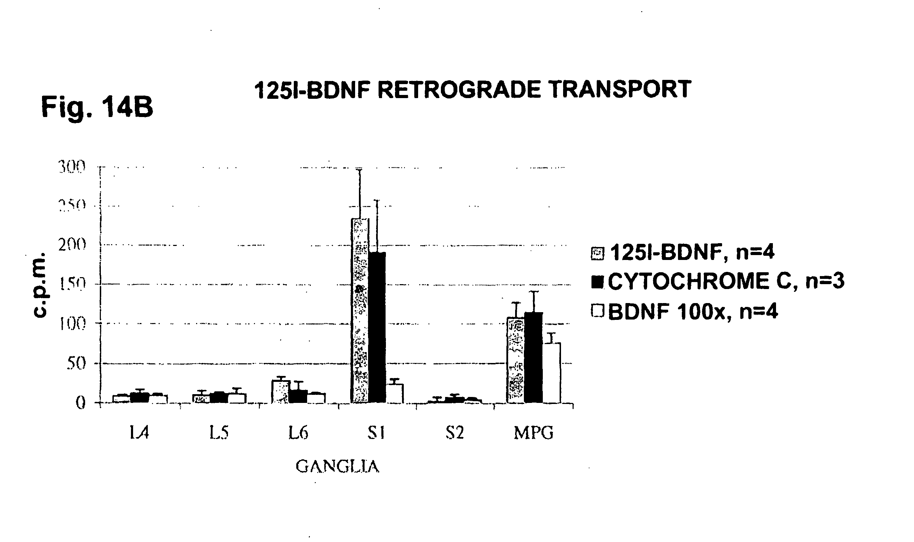

**Figure 14.** Quantification and specificity of retrograde transport of ¹²⁵I-NGF, -BDNF and -NT-3 into adult MPG and different DRGs. Iodinated neurotrophins were injected into the penis alone with 100 fold excess of unlabeled neurotrophine or cytochrome.

### REFERENCE LIST

Airaksinen, M.S. et al Mol Cell Neurosci 13 (1999) 313-325
Andersson, K.E. and Wagner, G. Physiol Rev 75 (1995) 191-236.
Arenas, E. et al. Neuron 15 (1995) 1465-1473.
Arumäe, U. et al. J Cell Biol 122 (1993) 1053-1065.
Baloh, R.H. et al. Neuron 18 (1997) 793-802.
Baloh, R.H. et al. Neuron 21 (1998) 1291-1302.
Barbacid, M. Curr Opin Cell Biol 7. (1995) 148-155.
Batra, A.K. & Lue, T.F. (1991) in Impotence: Diagnosis and Management of Male Erectile Dysfunction, eds. Kirby, R.S., Carson, C.C., Webster, G.D. (Butterworth Heinemann, London), pp. 19-26.
Bolton, A.E. and Hunter, W.M. Biochem J 133 (1973) 529-539.
Buj-Bello, A. et al. Neuron 15 (1995) 821-828.
Burnett, A.L. et al. Science, 257 (1992) 401-403.
Burnett, A.L. (1995) Biol. Reprod. 52, 485-489.
Cacalano, G. et al. Neuron 21 (1998) 53-62.
Canzian, F. et al. (1995) Mamm. Genome 6, 433-435.
Chomczynski, P. and Sacchi, N. Ana Biochem 162 (1987) 156-159.
Dail, W.G. (1993) in Nervous Control of the Urogenital System. ed. Maggi, C.A. (Harwood Academic Publishers, Chur), pp. 69-101
Dail, W.G. Microsc Res Tech 35 (1996) 95-106.
Dail, W.G. et al. Cell Tissue Res 282 (1995) 109-116.
Dail, W.G. et al. (1989) J. Auton. Nerv. Syst. 28 , 251-257.
Davies, A.M. J Neurobiol 25 (1994) 1334-1348.
de Groat, W.C. and Booth, A.M. In C.A. Maggi (Ed.), Nervous Control of the Urogenital System, Harwood Academic Publishers, Chur, Switzerland, 1993, pp. 467-524.
Ding, Y. et al. (1993) Neurosci. Lett. 164, 187-189.
Eardley, I.K. In R.S.C.C.W.G.D. Kirby (Ed.), Impotence: Diagnosis and Management of Male Erectile Dysfunction, Butterworth Heinemann, 1991
Ebendal, T. In R.A. Rush (Ed.), Nerve Growth Factors, John Wiley and Sons, Ltd., Chichester, UK, 1989, pp. 81-93.
Ebendal, T. et al. J Neurosci Res 40 (1995) 276-284.
Enomoto, H. et al. Neuron, 21 (1998) 317-324.
Ermisch, B. and Schwechheimer, K. Clin Neuropath 14 (1995) 130-136.
Griffin, W.S. et al. J Neurochem 65 (1995) 228-233.
Golden, J.P. et al. (1998) J. Comp. Neurol. 398, 139-150.
Haimoto, H. et al Lab Invest 57 (1987) 489-498.
Harper, S.J. et al. (1999) Exp. Neurol. 155, 327-330.
Hefti, F. Annu Rev Pharmacol Toxicol 37 (1997) 239-267.
Henderson, C.E. Curr Opin Neurobiol 6 (1996) 64-70.
Henderson, C.E. et al. Science, 266 (1994) 1062-1064.
Heuckeroth, R.O. et al. Neuron 22 (1999) 253-263.
Hiltunen, J.O. et al. Circ Res 79 (1996) 930-939.
Horger, B.A. et al. (1998) J. Neurosci. 18, 4929-4937.
Ishii, D.N. (1993) in Neurotrophic Factors, eds. Loughlin, S.E. & Fallon, J.H. (Academic Press, London), pp. 415-442.
Jing, S. et al. (1996) Cell 85, 1113-1124.
Jing, S. et al. J Biol Chem 272 (1997) 33111-33117.
Jung, G.-W. et al. (1998) J. Urol. (Baltimore) 160, 1899-1904.
Keast, J.R. and de Groat, W.C. J Comp Neurol 288 (1989) 3877-400
Klein, R. et al. (1990). Cell 61, 647-656.
Klein, R.D. et al. (1997) Nature (London) 387, 717-721.
Klinge, E. & Sjöstrand, N.O. (1994) in Handbook of Experimental Pharmacology, Vol 111, eds. Szekeres, L. & Gy Papp, J. (Springer, Heidelberg), pp. 533-573.
Kotzbauer, P.T. et al. Nature, 384 (1996) 467-470.
Lamballe, F. et al. (1993) EMBO Journal 12, 3083-3094.
Lapchak, P.A. et al. Exp Neurol 145 (1997) 309-321.
Leader, M. et al. Histopathology, 11 (1987) 63-72.
Lin, L.F. et al. Science, 260 (1993) 1130-1132.
Maisonpierre, P.C. et al. (1991). Genomics 10, 558-568.
Marchalonis, J.J. (1969) Biochem. J. 113, 299-305.
Merlio, J.P. et al. (1992) Neuroscience 51, 513-532.
Middlemas, D.S. et al (1991). Mol Cell Biol 11, 143-153.
Milbrandt, J. et al. Neuron, 20 (1998) 245-253.
Moore, M.W. et al. Nature, 382 (1996) 76-79.
Naveilhan, P. et al. (1998) Proc. Natl. Acad. Sci. U. S. A. 95, 1295-1300.
Nishino, J. et al. Neuron 23 (1999) 725-736
Oppenheim, R.W. et al. Nature 373 (1995) 344-346.
Pichel, J.G. et al. Cold Spring Harbor Symposia on Quantitative Biology, 61 (1996) 445-457.
Pirvola, U. et al (1994). Hear Res 75, 131-144.
Pirvola, U. et al. (1992). Proc Natl Acad Sci USA 89, 9915-9919.
Quinlan, D.M. et al. J Urol 141 (1989) 656-661.
Reeben, M. et al. (1998) Neuroscience 83, 151-159.
Rosenthal, A. (1999) Neuron 22, 201-203.
Rossi, J. et al. (1999) Neuron 22, 243-252.
Sanchez, M.P. et al. Nature, 382 (1996) 70-73.
Sanicola, M. et al. Proc Natl Acad Sci USA 94 (1997) 6238-6243.
Sariola, H. et al. (1994). J Embryol Exp Morph
Shelton, D.L. & Reichardt, L.F. (1986) J. Cell Biol. 102, 1940-1948.
Skalli, O. et al. J Cell Biol 103 (1986) 2787-2796.
Skaper, S.D. et al. MolCell Neurosci 12 (1998) 179-193.
Suvanto, P. et al. Hum Mol Genet 6 (1997) 1267-1273.
Te, A.E. et al. (1994) J. Urol. (Baltimore) 152, 2167-2172.
Thompson, R.J. et al. Brain Res 278 (1983) 224-228.
Tomac, A. et al. Nature 73 (1995) 335-339.
Trupp, M. et al. J Cell Bioll30 (1995) 137-148.
Trupp, M. et al. (1998) Mol. Cell. Neurosci. 11, 47-63.
Tso, J.Y. et al.. Nucl Acids Res 13 (1985) 2485-2502.
Tsoulfas, P. et al. (1993). Neuron 10, 975-990.
Valenzuela, D.M. et al. (1993). Neuron 10, 963-974.
Ylikoski, J. et al. (1993). Hear Res 65, 69-78.
Yu, T. et al (1998) J. Neurosci. 18, 4684-4696.
Walsh, P.C. and Donker, P.J Urol 128 (1982) 492-497.
Widenfalk, J. et al. (1997) J. Neurosci. 17, 8506-8519.
Wilkinson, D.G. and Green, J. In A.J. Copp and D.L. Cockroft (Eds.), Postimplantation Mammalina Embryos: A Practical Approach, IRL Press., Oxford, 1990, pp. 155-171.
Yan, Q. et al. Nature, 373 (1995) 341-344.

## Claims

1. The use of a neurotrophic factor, which is a neurturin (NTN), a glial cell-derived neurotrophic factor (GDNF), or conservatively substituted variants thereof, alone or in any combination, or nucleotide sequences encoding said neurotrophic factors for manufacturing a medical product for treating penile erectile dysfunctions.

2. The use according to claim 1, **characterized in that** neurotrophic factor is NTN.

3. The use according to claim 1, **characterized in that** neurotrophic factor is GDNF.

4. The use according to claim 1, **characterized in that** the penile erectile dysfunction is caused by acute penile trauma, penile surgery, prostatic surgery, bladder surgery or other pelvic surgery.

5. The use according to claim 1**, characterized in that** the medical product is intracavernosally administrable.

6. The use according to claim 1, **characterized in that** the medical product in addition to the neurotrophic factor comprises at least one optional pharmaceutically acceptable auxiliary agent which is compatible with the route of administration.

## Patentansprüche

1. Verwendung eines neurotrophen Faktors, der ein Neurturin (NTN), ein von Gliazellen abgeleiteter neurotropher Faktor ("glial cell-drived neurotrophic factor") (GDNF) ist, oder konservativ substituierter Varianten davon, alleine oder in beliebiger Kombination, oder von Nukleotidsequenzen, die für die neurotrophen Faktoren codieren, zur Herstellung eines medizinischen Produkts zur Behandlung peniler erektiler Dysfunktionen.

2. Verwendung nach Anspruch 1, **dadurch gekennzeichnet, dass** der neurotrophe Faktor NTN ist.

3. Verwendung nach Anspruch 1, **dadurch gekennzeichnet, dass** der neurotrophe Faktor GDNF ist.

4. Verwendung nach Anspruch 1, **dadurch gekennzeichnet, dass** die penile erektile Dysfunktion durch akutes Penistrauma, Penisoperation, Prostataoperation, Blasenoperation oder eine andere Beckenoperation verursacht ist.

5. Verwendung nach Anspruch 1, **dadurch gekennzeichnet, dass** das medizinische Produkt intrakavernös verabreichbar ist.

6. Verwendung nach Anspruch 1, **dadurch gekennzeichnet, dass** das medizinische Produkt zusätzlich zu dem neurotrophen Faktor wenigstens einen optionalen, pharmazeutisch verträglichen Hilfsstoff umfasst, der mit dem Verabreichungsweg kompatibel ist.

## Revendications

1. Utilisation d'un facteur neurotrophique, qui est la neurturine (NTN), d'un facteur neurotrophique dérivé des cellules gliales (GDNF), ou ses variantes conservativement substituées, seul ou en combinaison ou bien des séquences de nucléotides codant pour lesdits facteurs neurotrophiques pour la fabrication d'un produit médical pour le traitement des dysfonctionnements érectiles péniens.

2. Utilisation selon la revendication 1, **caractérisée en ce que** le facteur neurotrophique est NTN.

3. Utilisation selon la revendication 1, **caractérisée en ce que** le facteur neurotrophique est GDNF.

4. Utilisation selon la revendication 1, **caractérisée en ce que** le dysfonctionnement érectile pénien est provoqué par un traumatisme pénien aigu, une chirurgie pénienne, une chirurgie prostatique, une chirurgie de la vessie ou autre chirurgie pelvienne.

5. Utilisation selon la revendication 1, **caractérisée en ce que** le produit médical est administrable par voie intracaverneuse.

6. Utilisation selon la revendication 1, **caractérisée en ce que** le produit médical, en plus du facteur neurotrophique, comprend au moins un agent auxiliaire facultatif pharmaceutiquement acceptable qui est compatible avec la voie d'administration.
